(19) Europäisches Patentamt
European Patent Office
Office européen des brevets

(11) Publication number: 0 306 055 B1

(12) # EUROPEAN PATENT SPECIFICATION

(45) Date of publication of patent specification: **02.12.92** (51) Int. Cl.5: **C07C 275/54**, A01N 47/34

(21) Application number: **88114451.3**

(22) Date of filing: **05.09.88**

(54) **N-(halobenzoyl)-N'-2-halo-4-[1,1,2-trifluoro-2-(trifluoro-methoxy)ethoxy]-phenyl-ureas with insecticide activity.**

(30) Priority: **04.09.87 IT 2179487**

(43) Date of publication of application:
**08.03.89 Bulletin 89/10**

(45) Publication of the grant of the patent:
**02.12.92 Bulletin 92/49**

(84) Designated Contracting States:
**AT BE CH DE ES FR GB LI NL SE**

(56) References cited:
**EP-A- 0 088 343**
**EP-A- 0 226 642**
**EP-A- 0 277 748**
**EP-A- 0 203 618**
**EP-A- 0 271 923**
**EP-A- 0 337 600**

(73) Proprietor: **ISTITUTO GUIDO DONEGANI S.p.A.**
**Via Caduti del Lavoro**
**I-28100 Novara(IT)**

(72) Inventor: **Massardo, Pietro, Dr.-Chem.**
**5, via Fra' Bartolomeo**
**I-20123 Milan(IT)**
Inventor: **Meazza, Giovanni, Dr.-Chem.**
**30, via Pastore**
**I-21047 Saronno Varese(IT)**
Inventor: **Bettarini, Franco, Dr.-Chem.**
**4/B, via Cadore**
**I-28100 Novara(IT)**
Inventor: **Castoro, Paolo**
**16, via Birago**
**I-13100 Vercelli(IT)**
Inventor: **Caprioli, Vincenzo, Dr.-Biol.**
**8, via Loriga**
**I-27028 S. Martino Siccomario Pavia(IT)**

(74) Representative: **Zumstein, Fritz, Dr. et al**
**Dr. F. Zumstein Dipl.-Ing. F. Klingseisen**
**Bräuhausstrasse 4**
**W-8000 München 2(DE)**

EP 0 306 055 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a class of insecticide compounds, and more precisely it relates to N-(halobenzoyl)-N′-2-halo-4-[1,1,2-trifluoro-2-(trifluoro-methoxy)ethoxy]-phenyl-ureas endowed with a high insecticide activity, suitable for being used in the agrarian, forestal, civil and veterinary field in the fight against the infestations by insects. These compounds are particularly active against eggs and larvae of insects.

In European Patent Application No. 203,618 in the same Applicant's name, insecticide compounds are disclosed, which are derivatives of 1-benzoyl-3-aryl-ureas, having the general formula:

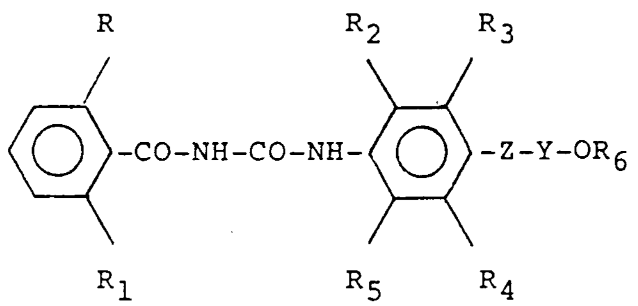

(I)

wherein:

| | |
|---|---|
| R | = Cl, F; |
| $R_1$ | = H, Cl, F; |
| $R_2$ and $R_5$, | which can be either equal to, or different from each other, are: H, halogen ($C_1$-$C_4$)-alkyl; |
| $R_3$ and $R_4$, | which can be either equal to, or different from, each other, are: H, halogen, alkyl, haloalkyl, halo-alkenyl-oxy or alkynyl; |
| Z | = O, S, or an $NR_7$ group, wherein $R_7$ is either ($C_1$-$C_3$)-alkyl or H; |
| Y | = ($C_1$-$C_4$)-alkylene, halo-ethylene or halo-ethenyl; |
| $R_6$ | = ($C_1$-$C_4$)-alkyl; ($C_1$-$C_4$)-halo-alkyl; ($C_3$-$C_4$)-alkenyl; ($C_3$-$C_4$)-halo-alkenyl; ($C_3$-$C_4$)-cycloalkyl; ($C_3$-$C_4$)-halo-cycloalkyl; or ($C_3$-$C_4$)-halo-cycloalkenyl. |

During its studies on the compounds disclosed in the above mentioned European patent application, the present Applicant has found that, among the compounds having formula (I), a particular class of compounds, N-(halobenzoyl)-N′-2-halo-4-[1,1,2-trifluoro-2-(trifluoro-methoxy)ethoxy]-phenyl-ureas, exist, which are surprisingly endowed with a decidedly higher insecticide activity than of analogous compounds.

The object of the present invention is therefore the class of compounds N-(halobenzoyl)-N′-2-halo-4-[1,1,2-trifluoro-2-(trifluoro-methoxy)ethoxy]-phenyl-ureas having the formula:

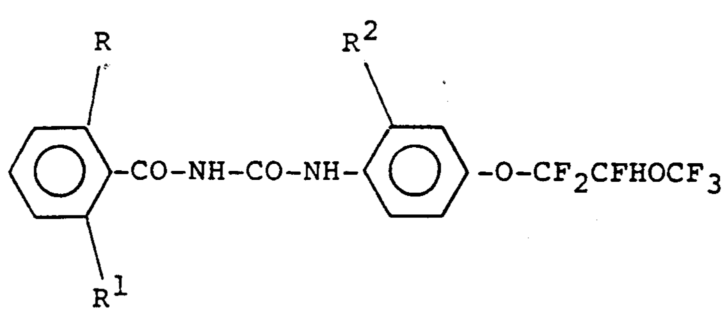

(II)

wherein:

| | |
|---|---|
| R, $R^1$, | which can be either equal to, or different from, each other, are H, F or Cl; at least one of R and $R^1$ is either F or Cl; |
| $R^2$ | is either F or Cl. |

The compounds of formula (II) are endowed with a particularly high insecticide activity, unexpectedly higher than the insecticide activity displayed by the analogous compounds of formula (I) which have been

disclosed in the above mentioned European patent application.

The compound having formula (II) can be used as such, or in the form of suitable compositions, in the fight against the infestations by noxious insects.

Further objects of the present invention are hence the use of the compounds of formula (II) in the fight against the insects, and the insecticide compositions which contain the compound of formula (II) as the active principle.

The preparation of the compounds of formula (II) is carried out by means of the reaction, as already generally described in the above mentioned European patent application, between a benzoyl isocyanate and an aromatic amine, in particular by means of the reaction between a halo-benzoyl-isocyanate (III) and a 2-halo-4-[1,1,2-trifluoro-2-(trifluoromethoxy)ethoxy]-aniline (IV), according to the following equation:

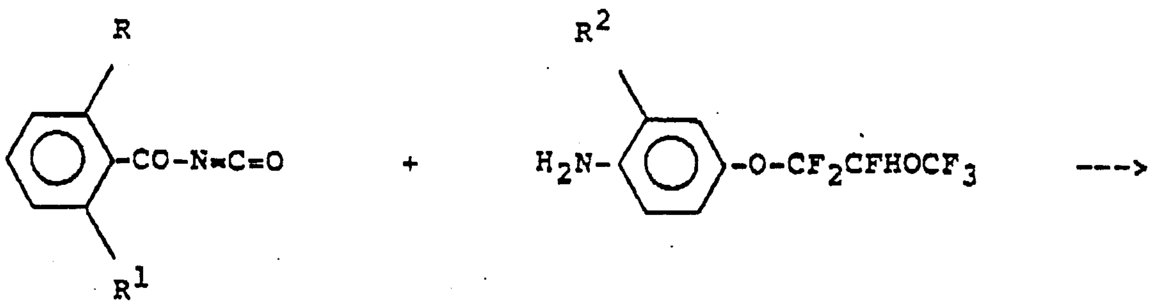

(III) (IV)

---> (II)

wherein R, $R^1$ and $R^2$ have the same meanings as hereinabove stated for formula (II).

The reaction does not require the presence of catalysts, and is carried out in an inert solvent and at a temperature comprised between 0 °C and the boiling temperature of the mixture.

The benzoylisocyanates having formula (III) are known compounds, and can be prepared by per se known methods. The amines of formula (IV) can be prepared according to known methods, and, more precisely, they can be prepared according to the methods as disclosed in the hereinabove mentioned European patent application, preferably by reacting the sodium or potassium salt of a 3-halo-4-aminophenol (V) of formula:

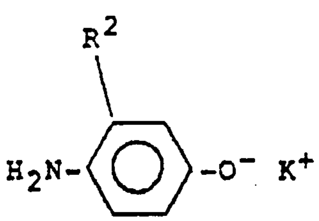

(V)

wherein $R^2$ has the same meaning as hereinabove stated for formula (II), with perfluorovinyl-perfluoromethyl-ether (VI), having the formula

$CF_2 = CF\text{-}OCF_3$

in polar aprotic solvents, at temperatures comprised within the range of from 0 °C to room temperature.

The ethers of formula (VI) can be prepared according to methods known from the prior art, as described, e.g., in J. Org. Chem., 48, 242 (1983).

An alternative route for the synthesis of the compounds of formula (II) consists in reacting a halobenzamide (VII) with a 2-halo-4-[1,1,2-trifluoro-2-(trifluoromethoxy)-ethoxy]-phenyl-isocyanate (VIII) according to the equation:

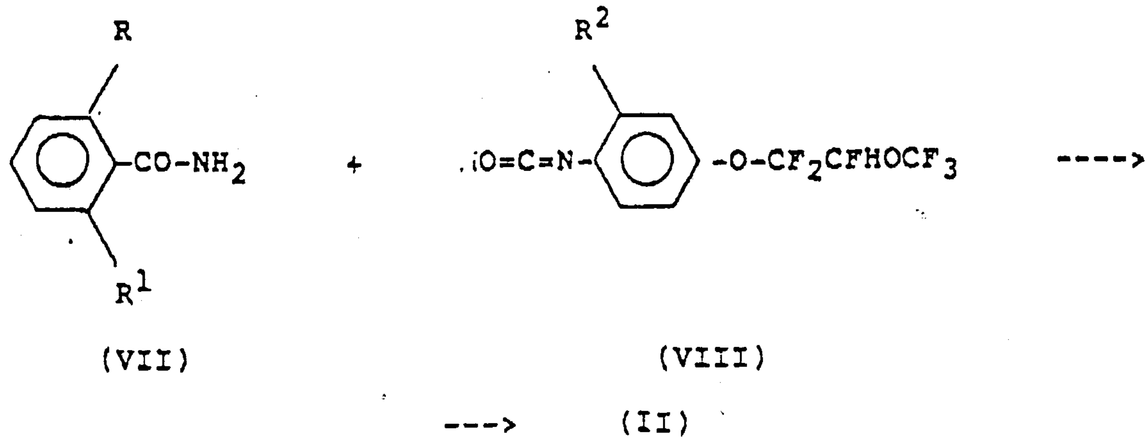

(VII) (VIII)

(II)

wherein R, $R^1$ and $R^2$ have the same meanings as hereinabove stated for formula (II).

Such a reaction is carried out under analogous conditions to such conditions as disclosed for the reaction between benzoyl-isocyanates of formula (III) and the amines of formula (IV).

As it has been previously mentioned, the compounds of formula (II) are endowed with a high insecticide activity, with a particularly high activity being above all displayed against insect eggs and larvae.

Among these, in particular, by means of the compounds having formula (II), the insects belonging to the Lepidoptera, Diptera, Coleoptera orders can be fought in particular.

These orders comprise a large number of species important for their noxiousness in the agrarian, forestal, civil and veterinary field. Thus, the compounds of formula (II) are fit for many uses, such as, e.g. the defense of agrarian cultivations against the infestations by phytophagous insects, the protection of sites infested by mosquitos and flies, the protection of breeding-cattle against some cattle parasites, and so forth.

For practical uses, the compounds of formula (II) can be used as such or, more adequately, in the form of compositions containing, besides one or more of the compounds of the above said formula (II) as the active principle, also solid or liquid inert carriers and, optionally, further additives. According to the usual formulative practice, the compositions can be supplied as wettable powders, emulsifiable concentrates, etc.

The amount of active principle constituted by one or more compounds of formula (II) in the compositions may vary within a wide range (from 1 to 95% by weight), depending on the type of composition and on the use it is intended for.

Furthermore, the amount of active substance to be distributed for the insecticide treatments will depend on various factors, such as, e.g., the type of infestation, the environment wherein the infestation is occurring (agrarian cultivations, ponds or watercourses, organic substrates of various kinds), the type of composition used, climatic and environmental factors, available application means, and so forth. In general, amounts of active substance comprised within the range of from 0.01 to 1 kg/ha are sufficient for a good disinfestation.

The following examples are given to the purpose of better illustrating the invention, without limiting it in any way.

EXAMPLE 1

Preparation of N-(2,6-difluorobenzoyl)-N′-2-fluoro-4-[1,1,2-trifluoro-2-(trifluoromethoxy)ethoxy]-phenyl-urea (Compound No. 1)

No. 1)

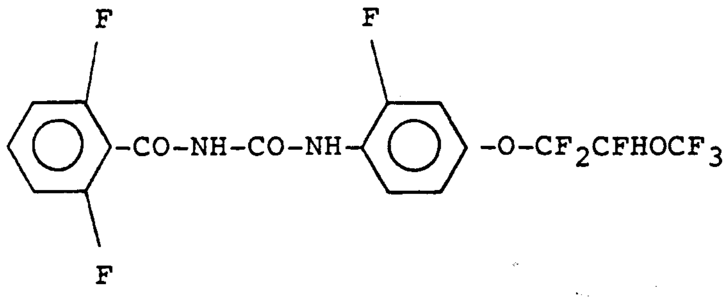

To a four-necked flask of 500 ml of capacity, fitted with condenser, thermometer, dropping funnel and mechanical stirring means, 23 g of 2-fluoro-4-[1,1,2-trifluoro-2-(trifluoromethoxy)ethoxy]-aniline dissolved in 200 ml of anhydrous ethyl ether is charged under nitrogen.

14.3 g of 2,6-difluoro-benzoyl-isocyanate dissolved in 50 ml of anhydrous ethyl ether is then added dropwise at room temperature.

The reaction mixture is refluxed with stirring for 1 hour; it is then cooled to 0 ° C, is filtered under nitrogen, the precipitate is washed with cold n-hexane and is finally dried under nitrogen.

32.4 g (87%) of benzoyl-urea having a melting point of 139 ° C is finally obtained.

EXAMPLE 2

By starting from the anilines disclosed in following Example 3, and by operating under analogous conditions to those as disclosed in example 1, by using 2,6-difluoro-benzoyl-isocyanate, the following compound:

- Compound No. 2: N-2,6-difluorobenzoyl-N′-2-chloro-4-[1,1,2-trifluoro-2-(trifluoromethoxy)-ethoxy]-phenyl-urea:

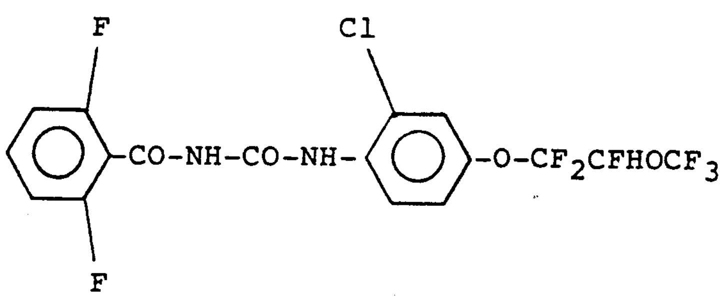

(melting point [m.p.] = 147 ° C)

was prepared, whilst by using 2-chlorobenzoyl-isocyanate, the following compounds were prepared:

- Compound No. 3: N-2-chlorobenzoyl-N′-2-fluoro-4-[1,1,2-trifluoro-2-(trifluoromethoxy)-ethoxy]-phenyl-urea:

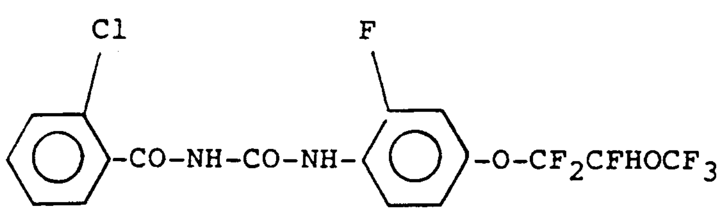

m.p. = 127 ° C

- Compound No. 4: N-2-chlorobenzoyl-N′-2-chloro-4-[1,1,2-trifluoro-2-(trifluoromethoxy)-ethoxy]-phenyl-urea:

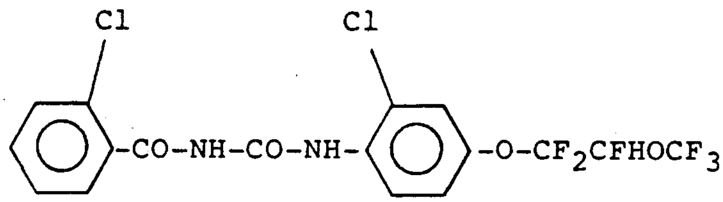

m.p. = 116 °C.

Example 3

Preparation of the intermediate Anilines

To a three-necked flask of 100 ml of capacity fitted with thermometer, pierceable cap for drawings, connection to a supply of perfluorovinyl-perfluoromethyl-ether gas and magnetic stirrer, 1.27 g of 3-fluoro-4-amino-phenol dissolved in 40 ml of a mixture constituted by toluene and dimethyl-sulphoxide in the ratio of 8:2 is charged under nitrogen. 100 mg of finely ground KOH at 85% is added, the system is evacuated and from the gas supply 1.66 g of perfluorovinyl-perfluoromethyl-ether is added.

The reaction is let proceed at room temperature for one hour, the reaction mixture is then poured in 100 ml of water and is extracted with ethyl ether. The ethereal phase is thoroughly dried over sodium sulphate and is concentrated; 2.8 g of 2-chloro-4-[1,1,2-trifluoro-2-(trifluoromethoxy)-ethoxy]-aniline is obtained.

The proton nuclear magnetic resonance spectrum ($^1$H-N.M.R.) has the following characteristics:

$^1$H-N.M.R. ($CDCl_3$): 6.9-6.4 $\delta$ (m, 3H, aromatic); 6.25-5.35 $\delta$ (dt, 1H, -CFH-); 3.55 $\delta$ (s, 2H, $-NH_2$).

By operating under the above disclosed conditions, but using 3-chloro-4-amino-phenol and $CF_2 = CF-O-CF_3$, 2-chloro-4-[1,1,2-trifluoro-2-(trifluoromethoxy)-ethoxy]-aniline was obtained:

$^1$H-N.M.R.($CDl_3$): 7.3-6.7 $\delta$ (m, 3H, aromatic); 6.6-5.7 $\delta$ (dt, 1H, -CFH); 3.7 $\delta$ (bs, 2H, $-NH_2$).

The abbreviations in the spectra have the following meaning:

| | | |
|---|---|---|
| s | = | singlet; |
| m | = | multiplet, or unresolved complex signal; |
| t | = | triplet; |
| d | = | doublet; |
| b (broad) | = | broad signal; |

Example 4

Determination of the Insecticide Activity

Test 1

Immediate Residual Activity on larvae of Spodoptera Immediate Residual Activity on larvae of Spodoptera littoralis (Lepidoptera)

Tobacco leaves are treated by mechanical spraying with a water-acetonic solution of the product under test at 10% by volume of acetone, and containing a surfactant.

After the complete evaporation of the solvents, the leaves are infested with second-age larvae of the lepidopteran.

The infested leaves are stored in a suitably conditioned room throughout the test.

Infested and stored in a similar way are tobacco leaves treated with the water-acetonic solution (at 10%) only, and with the surfactant, for use for comparison purposes, as the control group.

Ten days after the infestation, and after renewing the treated substrate at least once, the dead larvae are counted, as compared to the control group.

Test 2

Activity of Larvae of Aedes aecypti (Diptera)

Spring water (297 ml) is mixed with an acetonic solution (3 ml) of the product under test, at a suitable concentration.

Into the resulting solution are introduced 25 4-day-old larvae of the mentioned dipteran, which are suitably fed during the whole observation time period. As the control group, other larvae are introduced into a water-acetonic solution (297 ml of spring water, 3 ml of acetone), without any active substances.

The number of dead larvae and pupae, or of adult insects normally emerged from the cocoon is noted every 2-3 days, until the end of the insects emergence from the cocoons of the control group.

The activity of the product being tested is expressed as the percentage of individuals dead relatively to the total number of treated individuals, possibly corrected according to Abbot (in case of partial death in the control group).

In following Table 1, the data relating to the insecticide activity at the indicated doses, expressed as parts per million of active substance, is reported.

For both tests, 4 repeated tests for each dosage/thesis combination were taken into consideration, and a number of tests were carried out at different times, which is regarded as sufficient in order to give statistic significance to small differences in efficacy (with the dosage being the same).

TABLE 1

| Insecticide Activity | | | | | | |
|---|---|---|---|---|---|---|
| Compound No. | 1 | | 2 | | 3 | |
| | Dosage ppm | Death rate % | Dosage ppm | Death rate % | Dosage ppm | Death rate % |
| Test No. 1 | 0.005<br>0.001<br>0.0005 | 100<br>97<br>64 | 0.01<br>0.005<br>0.001 | 100<br>95<br>75 | 0.01<br>0.005<br>0.001 | 100<br>98<br>75 |
| Test No. 2 | 0.0002<br>0.00002<br>0.000002 | 100<br>88<br>80 | n.d. | | 0.0002<br>0.00002<br>0.000002<br>0.0000002 | 100<br>90<br>77<br>71 |

Continuation of TABLE 1

| Insecticide Activity | | | | | | | | |
|---|---|---|---|---|---|---|---|---|
| Compound No. | 4 | | Ref. 1 | | Ref. 2 | | Ref. 3 | |
| | Dosage ppm | Death rate % | Dosage ppm | Death rate % | Dosage ppm | Death rate % | Dosage ppm | Death rate % |
| Test No. 1 | 0.01<br>0.005<br>0.001 | 100<br>85<br>77 | 1.0<br>0.5<br>0.1 | 100<br>70<br>35 | 1.0<br>0.5<br>0.1<br>0.05 | 100<br>95<br>78<br>40 | 0.1<br>0.05<br>0.01<br>0.005 | 100<br>93<br>78<br>31 |
| Test No. 2 | n.d. | | 0.2<br>0.02 | 100<br>25 | 0.02<br>0.002<br>0.0002 | 100<br>86<br>41 | n.d. | |

As the reference 1, the compound:
N-2,6-difluorobenzoyl-N′-4-[1,1,2-trifluoro-2-(trifluoromethoxy)ethoxy]-phenyl urea was taken.

As the reference 2, the compound:
N-2,6-difluorobenzoyl-N′-4-[1,1,2-trifluoro-2-(perfluoroethoxy)ethoxy]-phenyl-urea was taken.

As the reference 3, the compound:
N-2-(chlorobenzoyl)-N′-3,5-dichloro-4-[1,1,2-trifluoro-2-(trifluoromethoxy)ethoxy]-phenyl-urea was taken.

Said compounds are comprised in European patent application No. 203 618.

## Claims

### Claims for the following Contracting States : AT, BE, CH, DE, FR, GB, LI, NL, SE

**1.** N-(halobenzoyl)-N′-2-halo-4-[1,1,2-trifluoro-2-(trifluoromethoxy)ethoxy]-phenyl-ureas having the formula:

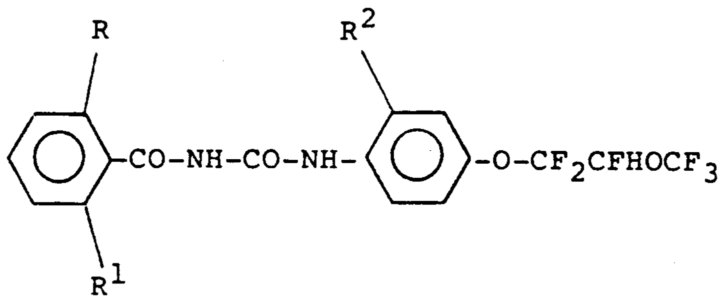

wherein:

R, $R^1$, which can be either equal to, or different from, each other, are H, F or Cl; at least one of R and $R^1$ is either F or Cl;

$R^2$ is either F or Cl.

**2.** The compound:
N-(2,6-difluorobenzoyl)-N′-2-fluoro-4-[1,1,2-trifluoro-2-(trifluoromethoxy)-ethoxy]-phenyl-urea.

**3.** The compound:
N-2,6-difluorobenzoyl-N′-2-chloro-4-[1,1,2-trifluoro-2-(trifluoromethoxy)-ethoxy]-phenyl-urea.

**4.** The compound:
N-2-chlorobenzoyl-N′-2-fluoro-4-[1,1,2-trifluoro-2-(trifluoromethoxy)-ethoxy]-phenyl-urea.

**5.** The compound:
N-2-chlorobenzoyl-N′-2-chloro-4-[1,1,2-trifluoro-2-(trifluoromethoxy)-ethoxy]-phenyl-urea.

**6.** Method for fighting infestations caused by noxious insects, consisting in distributing on the infested area an effective amount of one or more of the compounds of claim 1, either as such, or in the form of a suitable composition.

**7.** Insecticide compositions containing as the active ingredient one or more of the compounds of claim 1, together with solid or liquid inert carriers and, optionally, other additives.

### Claims for the following Contracting State : ES

**1.** Method for fighting infestations caused by noxious insects, consisting in distributing on the infested area an effective amount of one or more
N-(halobenzoyl)-N'-2-halo-4-[1,1,2-trifluoro-2-(trifluoromethoxy)ethoxy]-phenyl-ureas having the formula:

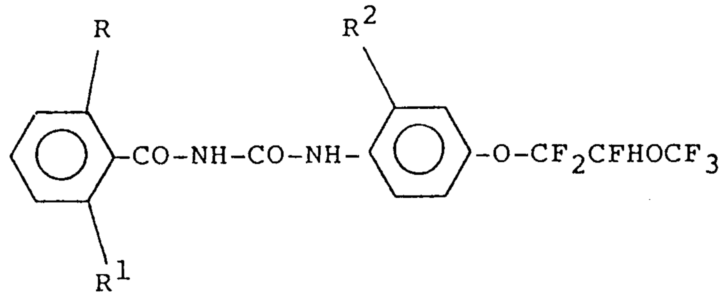

wherein:

R, $R^1$, which can be either equal to, or different from, each other, are H, F or Cl; at least one of R and $R^1$ is either F or Cl;

$R^2$ is either F or Cl,

either as such, or in the form of a suitable composition.

2. Method according to claim 1 consisting in distributing the compound: N-(2,6-difluorobenzoyl)-N'-2-fluoro-4-[1,1,2-trifluoro-2-(trifluoromethoxy)-ethoxy]-phenyl-urea.

3. Method according to claim 1 consisting in distributing the compound: N-2,6-difluorobenzoyl-N'-2-chloro-4-[1,1,2-trifluoro-2-(trifluoromethoxy)-ethoxy]-phenyl-urea.

4. Method according to claim 1 consisting in distributing the compound: N-2-chlorobenzoyl-N'-2-fluoro-4-[1,1,2-trifluoro-2-(trifluoromethoxy)-ethoxy]-phenyl-urea.

5. Method according to claim 1 consisting in distributing the compound: N-2-chlorobenzoyl-N'-2-chloro-4-[1,1,2-trifluoro-2-(trifluoromethoxy)-ethoxy]-phenyl-urea.

6. Insecticide compositions containing as the active ingredient one or more of the compounds of claim 1, together with solid or liquid inert carriers and, optionally, other additives.

**Patentansprüche**

**Patentansprüche für folgende Vertragsstaaten : AT, BE, CH, DE, FR, GB, LI, NL, SE**

1. N-(Halobenzoyl)-N'-2-halo-4-[1,1,2-trifluor-2-(trifluormethoxy)-ethoxy]-phenyl-harnstoffe der Formel

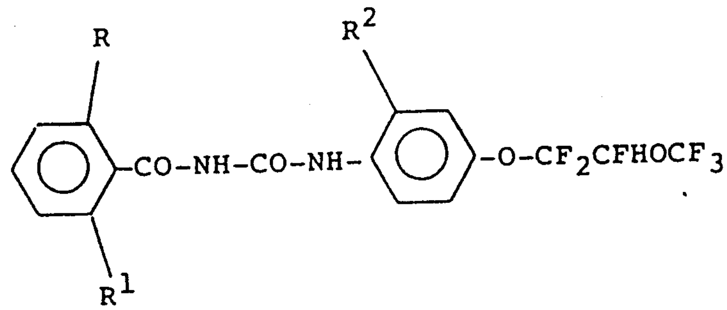

worin

R und $R^1$, die gleich oder voneinander verschieden sein können, jeweils für H, F oder Cl stehen; wobei zumindest eines von R und $R^1$ entweder F oder Cl ist;

$R^2$ entweder F oder Cl ist.

2. Die Verbindung: N-(2,6-Difluorbenzoyl)-N'-2-fluor-4-[1,1,2-trifluor-2-(trifluormethoxy)-ethoxy]-phenyl-harnstoff.

3. Die Verbindung:
N-2,6-Difluorbenzoyl-N'-2-chlor-4-[1,1,2-trifluor-2-(trifluormethoxy)-ethoxy]-phenyl-harnstoff.

4. Die Verbindung:
N-2-Chlorbenzoyl-N'-2-fluor-4-[1,1,2-trifluor-2-(trifluormethoxy)-ethoxy]-phenyl-harnstoff.

5. Die Verbindung:
N-2-Chlorbenzoyl-N'-2-chlor-4-[1,1,2-trifluor-2-(trifluormethoxy)-ethoxy]-phenyl-harnstoff.

6. Verfahren zur Bekämpfung von durch schädliche Insekten verursachtem Befall , darin bestehend, daß man auf die befallene Fläche eine wirksame Menge einer oder mehrerer der Verbindungen gemäß Anspruch 1 entweder als solche oder in Form einer geeigneten Zusammensetzung verteilt.

7. Insektizide Zusammensetzungen, enthaltend als Wirkstoff eine oder mehrere Verbindungen gemäß Anspruch 1 zusammen mit festen oder flüssigen, inerten Trägern und gegebenenfalls anderen Additiven.

**Patentansprüche für folgenden Vertragsstaat : ES**

1. Verfahren zur Bekämpfung von durch schädliche Insekten verursachtem Befall, darin bestehend, daß man auf die befallene Fläche eine wirksame Menge eines oder mehrerer N-(Halobenzoyl)-N'-2-halo-4-[1,1,2-trifluor-2-(trifluormethoxy)-ethoxy]-phenyl-harnstoffe der folgenden Formel

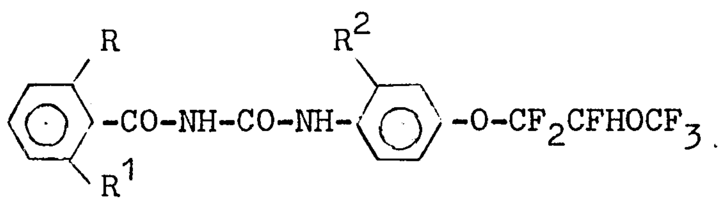

worin
R und $R^1$, die gleich oder voneinander verschieden sein können, jeweils für H, F oder Cl stehen; wobei zumindest eines von R und $R^1$ entweder F oder Cl ist;
$R^2$ entweder F oder Cl ist,
entweder als solche oder in Form einer geeigneten Zusammensetzung verteilt.

2. Verfahren gemäß Anspruch 1, darin bestehend, daß man die Verbindung N-(2,6-Difluorbenzoyl)-N'-2-fluor-4-[1,1,2-trifluor-2-(trifluormethoxy)-ethoxy]-phenyl-harnstoff verteilt.

3. Verfahren gemäß Anspruch 1, darin bestehend, daß man die Verbindung N-2,6-Difluorbenzoyl-N'-2-chlor-4-[1,1,2-trifluor-2-(trifluormethoxy)-ethoxy]-phenyl-harnstoff verteilt.

4. Verfahren gemäß Anspruch 1, darin bestehend, daß man die Verbindung N-2-Chlorbenzoyl-N'-2-fluor-4-[1,1,2-trifluor-2-(trifluormethoxy)-ethoxy]-phenyl-harnstoff verteilt.

5. Verfahren gemäß Anspruch 1, darin bestehend, daß man die Verbindung N-2-Chlorbenzoyl-N'-2-chlor-4-[1,1,2-trifluor-2-(trifluormethoxy)-ethoxy]-phenyl-harnstoff verteilt.

6. Insektizide Zusammensetzungen, enthaltend als Wirkstoff eine oder mehrere Verbindungen gemäß Anspruch 1 zusammen mit festen oder flüssigen, inerten Trägern und gegebenenfalls anderen Additiven.

**Revendications**
**Revendications pour les Etats contractants suivants : AT, BE, CH, DE, FR, GB, LI, NL, SE**

1. N-(halobenzoyl)-N'-2-halo-4-[1,1,2-trifluoro-2-(trifluorométhoxy)éthoxy]-phénylurées de formule:

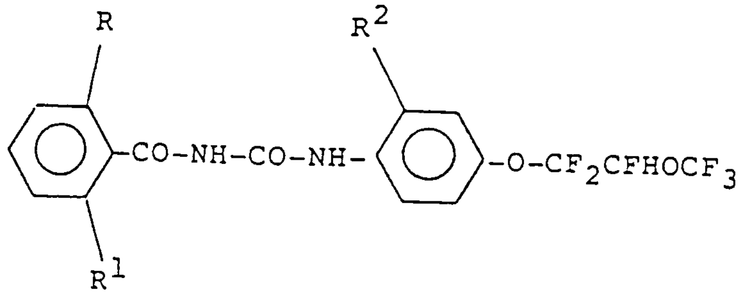

dans laquelle:

R et $R^1$, qui peuvent être identiques ou différents, sont H, F ou Cl, au moins un des groupes R et $R^1$ étant F ou Cl;

$R^2$ est F ou Cl.

**2.** Le composé consistant en N-(2,6-difluorobenzoyl)-N'-2-fluoro-4-[1,1,2-trifluoro-2-(trifluorométhoxy)-éthoxy]-phénylurée.

**3.** Le composé consistant en N-2,6-difluorobenzoyl-N'-2-chloro-4-[1,1,2-trifluoro-2-(trifluorométhoxy)-éthoxy]-phénylurée.

**4.** Le composé consistant en N-2-chlorobenzoyl-N'-2-fluoro-4-[1,1,2-trifluoro-2-(trifluorométhoxy)éthoxy]-phénylurée.

**5.** Le composé consistant en N-2-chlorobenzoyl-N'-2-chloro-4-[1,1,2-trifluoro-2-(trifluorométhoxy)éthoxy]-phénylurée.

**6.** Méthode de lutte contre les infestations causées par des insectes nuisibles, consistant en la distribution sur la zone infestée d'une quantité efficace d'un ou plusieurs des composés selon la revendication 1, soit tel quel, soit sous la forme d'une composition appropriée.

**7.** Compositions d'insecticide contenant en tant qu'ingrédient actif un ou plusieurs des composés de la revendication 1, avec des supports inertes solides ou liquides et, éventuellement, d'autres additifs.

**Revendications pour l'Etat contractant suivant : ES**

**1.** Méthode de lutte contre les infestations causées par des insectes nuisibles, consistant en la distribution dans la zone infestée d'une quantité efficace d'une ou plusieurs N-(halobenzoyl)-N'-2-halo-4-[1,1,2-trifluoro-2-(trifluorométhoxy)éthoxy]-phénylurées de formule:

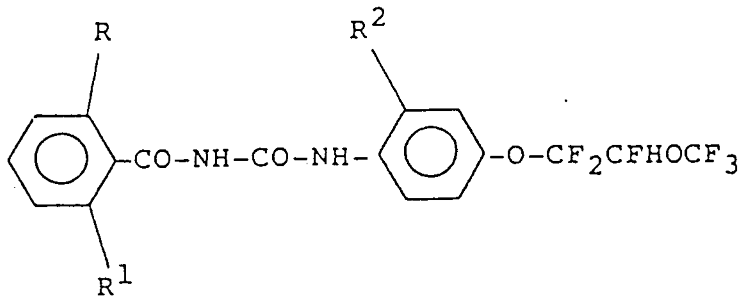

dans laquelle:

R et $R^1$, qui peuvent être identiques ou différents, sont H, F ou Cl, au moins un des groupes R et $R^1$ étant F ou Cl;

$R^2$ est F ou Cl,

soit tel quel, soit sous la forme d'une composition appropriée.

2. Méthode selon la revendication 1, consistant en la distribution du composé: N-(2,6-difluorobenzoyl)-N'-2-fluoro-4-[1,1,2-trifluoro-2-(trifluorométhoxy)éthoxy]-phénylurée.

3. Méthode selon la revendication 1, consistant en la distribution du composé: N-2,6-difluorobenzoyl-N'-2-chloro-4-[1,1,2-trifluoro-2-(trifluorométhoxy)éthoxy]-phénylurée.

4. Méthode selon la revendication 1, consistant en la distribution du composé: N-2-chlorobenzoyl-N'-2-fluoro-4-[1,1,2-trifluoro-2-(trifluorométhoxy)éthoxy]-phénylurée.

5. Méthode selon la revendication 1, consistant en la distribution du composé: N-2-chlorobenzoyl-N'-2-chloro-4-[1,1,2-trifluoro-2-(trifluorométhoxy)éthoxy]-phénylurée.

6. Compositions d'insecticide contenant en tant qu'ingrédient actif un ou plusieurs des composés selon la revendication 1, avec des supports inertes solides ou liquides et, éventuellement, d'autres additifs.